# EUROPEAN PATENT APPLICATION

(11) **EP 4 183 407 A1**
(43) Date of publication of application: **24.05.2023**
(21) Application number: 21842945.4
(22) Date of filing: 15.07.2021
(51) Int. Cl.: A61K 38/17, A61K 38/26, A61K 47/68, A61P 1/16, C07K 14/47, C07K 14/605

(54) **THERAPEUTIC USE OF GLUCAGON DERIVATIVE OR CONJUGATE THEREOF FOR LIVER DISEASE**

(30) Priority: 15.07.2020 KR 20200087684
(71) Applicant: Hanmi Pharm. Co., Ltd., Hwaseong-si, Gyeonggi-do 18536 (KR)
(72) Inventor: KIM, Jung Kuk, Hwaseong-si, Gyeonggi-do 18469 (KR); JO, Hyo Sang, Hwaseong-si, Gyeonggi-do 18469 (KR); KWON, Hyun Joo, Hwaseong-si, Gyeonggi-do 18469 (KR)
(74) Representative: Mewburn Ellis LLP
(86) International application number: PCT/KR2021/009124
(87) International publication number: WO 2022/015082

(57) **Abstract**

The present invention relates to the therapeutic use of a glucagon derivative or a conjugate thereof for liver disease.

## Description

### [Technical Field]

The present invention relates to the therapeutic use of a glucagon derivative or a conjugate thereof for liver disease.

### [Background Art]

The liver is one of the main body organs of animals and has various roles, such as lipid metabolism, detoxification, excretion of bile, storage of various nutrients, hematopoiesis or blood coagulation, and the control of a volume of circulating blood, *etc.* The liver regulates a large number of very diverse biochemical actions and is therefore essential for maintaining life. Representative examples of such liver-related diseases include steatosis, liver fibrosis, liver inflammation, cirrhosis, liver decompensation, hepatocellular carcinoma, cholestatic liver disease, *etc.*

Steatosis, a type of fatty liver in which fat is accumulated in liver cells due to excessive intake of fat, or endogenous increase in fat synthesis in the liver or decrease in the excretion of fats, causes inflammation and may tend to progress to other liver diseases such as non-alcoholic steatohepatitis.

In general, hepatitis, which causes inflammation of the liver, accounts for most of the liver diseases, and it is known that various liver diseases (liver fibrosis, cirrhosis, *etc.)* appear as hepatitis progresses. Depending on the aspect of hepatitis, it can be divided into acute hepatitis and chronic hepatitis, and depending on the causes, it can be divided into viral hepatitis, alcoholic hepatitis, and drug-induced hepatitis. Cholestasis liver disease is also assumed to be caused by an inflammatory disease.

Other liver diseases include liver fibrosis, liver cirrhosis, liver decompensation, liver cancer, *etc.,* and since such liver diseases are initially asymptomatic and are discovered only after considerable progress, the need for drug development is high because these are the leading cause of death not only in Korea but also worldwide.

Liver inflammation, known as a major cause of liver disease, is caused by activation of Kupffer cells, which are liver resident macrophages, due to various genetic and environmental factors. Activated Kupffer cells not only induce liver inflammation by secreting various inflammatory cytokines, but also secrete chemokines into the blood to recruit various immune cells, including blood monocytes and neutrophils, to the liver, thereby sustaining liver inflammation. This continuous inflammation of the liver causes damage to the liver tissue, which is known to further intensify the inflammation of the liver. It is known that this vicious cycle ultimately induces liver fibrosis by inducing activation of hepatic stellate cells. In order to treat liver inflammation, which is the cause of various liver diseases including liver fibrosis, mainly therapy to remove the cause or symptomatic therapy is performed at present. However, the symptoms of hepatitis are not discovered until the disease has progressed considerably, and it is quite difficult to control the progression of the disease by means of prevention or symptomatic treatment alone. Therefore, the need for the development of therapeutic drugs that can directly act on hepatitis is still high.

Meanwhile, glucagon is produced in the pancreas when blood glucose begins to drop due to drug treatment or disease, hormone or enzyme deficiency, *etc.* Glucagon signals the liver to break down glycogen and release glucose, which raises blood glucose to normal levels. In addition, until recently, it had been suggested that glucagon can be used as a therapeutic agent for metabolic syndrome including obesity by promoting appetite suppression, increase in energy expenditure in adipose tissue, and improvement in lipid metabolism in liver tissue, in addition to the effect of raising blood glucose. However, its use as a therapeutic agent has been limited due to its low solubility and precipitation at neutral pH.

Accordingly, glucagon derivatives with improved physical properties have been developed and used for the treatment of metabolic syndrome, *etc.* (US 2017-0360892).

### [Disclosure]

### [Technical Problem]

The development of drugs for effective prevention and treatment of liver diseases is required.

### [Technical Solution]

It is one object of the present invention to provide a composition including the peptide of General Formula 1 or a conjugate including the peptide, specifically a pharmaceutical composition for preventing or treating liver disease.

It is another object of the present invention to provide a method for preventing or treating liver disease, including administering the peptide of General Formula 1 above or a conjugate including the same, or a composition including the same to a subject in need thereof.

It is still another object of the present invention to provide the use of the peptide of General Formula 1 or a conjugate including the same, or a composition including the same in the preparation of a medicament for preventing or treating liver disease.

It is yet another object of the present invention to provide the use of the peptide of General Formula 1 or a conjugate including the same, or a composition including the same in the prevention or treatment of liver disease.

It is even another object of the present invention to provide a pharmaceutical composition for preventing or treating liver disease, including a peptide including any one of the amino acid sequences of SEQ ID NOS: 20, 22, 33, 37, and 38, or a conjugate including the same.

It is further another object of the present invention to provide a method for preventing or treating liver disease, including administering a peptide including any one of the amino acid sequences of SEQ ID NOS: 20, 22, 33, 37, and 38 or a conjugate including the same, or a composition including the same to a subject in need thereof.

It is still further another object of the present invention to provide the use of a peptide including any one of the amino acid sequences of SEQ ID NOS: 20, 22, 33, 37, and 38 or a conjugate including the same, or a composition including the same in the preparation of a medicament for preventing or treating liver disease.

It is still further another object of the present invention to provide the use of a peptide including any one of the amino acid sequences of SEQ ID NOS: 20, 22, 33, 37, and 38 or a conjugate including the same, or a composition including the same in the prevention or treatment of liver disease.

### [Advantageous Effects]

The peptide having activity on the glucagon receptor according to the present invention or a conjugate thereof can be effectively used for preventing or treating liver disease.

### [Brief Description of Drawings]

FIG. 1 is a diagram confirming the effect of the long-acting conjugate of the glucagon derivative of SEQ ID NO: 37 according to the present invention on hepatitis.
FIG. 2 is a diagram confirming the effect of the long-acting conjugate of the glucagon derivative of SEQ ID NO: 37 according to the present invention on liver fibrosis.
FIG. 3 is a diagram confirming the effect of the long-acting conjugate of the glucagon derivative of SEQ ID NO: 37 on reducing triglyceride in the liver according to the present invention.

### [Detailed Description of the Invention]

One aspect for implementing the present invention provides a composition including a peptide or a conjugate including the peptide, specifically a pharmaceutical composition for preventing or treating liver disease.

In one embodiment, the present invention relates to a pharmaceutical composition for preventing or treating liver disease, including a pharmaceutically acceptable excipient; and a peptide including the amino acid sequence of General Formula 1 below in a pharmaceutically effective amount:
Y-X2-QGTF-X7-SDYSKY-X14-D-X16-X17-R-X19-X20-X21-FVQWLMNT-X30 (General Formula 1, SEQ ID NO: 46)
wherein,
X2 is aminoisobutyric acid (Aib);
X7 is threonine (T), valine (V), or cysteine (C);
X14 is leucine (L) or cysteine (C);
X16 is glutamic acid (E) or serine (S);
X17 is lysine (K), arginine (R), or cysteine (C);
X19 is alanine (A) or cysteine (C);
X20 is lysine (K) or glutamine (Q);
X21 is aspartic acid (D) or glutamic acid (E); and
X30 is cysteine (C) or is absent (with the proviso that when the amino acid sequence of General Formula 1 is identical to SEQ ID NO: 1 or SEQ ID NO: 12, it is excluded).

In another embodiment, the pharmaceutical composition is characterized in that the peptide is in the form of a long-acting conjugate, and the long-acting conjugate is represented by Chemical Formula 1 below:

[Chemical Formula 1] X-L-F

with the proviso that X is a peptide including the amino acid sequence of General Formula 1 above;
L is a linker containing ethylene glycol repeating units;
F is an immunoglobulin Fc region; and
- represents a covalent bond between X and L, and L and F.

As the composition according to any one of the above-described embodiments, the liver disease is any one selected from the group consisting of steatosis, liver fibrosis, liver inflammation, liver cirrhosis, liver decompensation, hepatocellular carcinoma, and cholestasis liver disease.

As the composition according to any one of the above-described embodiments, the cholestasis liver disease is any one selected from the group consisting of primary biliary cirrhosis, primary sclerosing cholangitis, and a combination thereof.

As the composition according to any one of the above-described embodiments, the liver disease is caused by non-alcoholic steatohepatitis (NASH) or is accompanied thereby, but is not limited thereto.

As the composition according to any one of the above-described embodiments, the peptide includes an amino acid sequence selected from SEQ ID NOS: 20, 22, 23, 27, 33, 35, 37, 38, 40, 41, 42, and 44.

As the composition according to any one of the above-described embodiments, the peptide includes an amino acid sequence selected from SEQ ID NOS: 20, 22, 23, 27, 33, 37, 38, and 44.

As the composition according to any one of the above-described embodiments, the pharmaceutical composition reduces a liver inflammation score in the administered subject upon administration.

As the composition according to any one of the above-described embodiments, the pharmaceutical composition reduces hydroxyproline content in the administered subject upon administration.

As the composition according to any one of the above-described embodiments, the pharmaceutical composition reduces triglyceride content in the liver tissue in the administered subject upon administration.

As the composition according to any one of the above-described embodiments, the C-terminus of the peptide is amidated.

As the composition according to any one of the above-described embodiments, the formula weight of the ethylene glycol repeating unit moiety in L is in the range of 1 kDa to 100 kDa.

As the composition according to any one of the above-described embodiments, the structure of Chemical Formula 1 is represented by the structure of Chemical Formula 3 below: 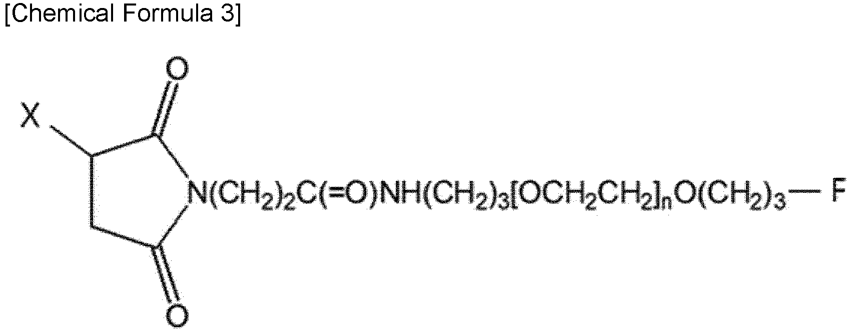 wherein, X and F are as defined in Chemical Formula 1.

As the composition according to any one of the above-described embodiments, the ethylene glycol repeating unit has a formula of [OCH₂CH₂]ₙ, wherein n is a natural number, and the average molecular weight of the [OCH₂CH₂]ₙ moiety in the peptide, for example, the number average molecular weight, is determined to be 1 kDa to 100 kDa.

As the composition according to any one of the above-described embodiments, the value of n is determined such that the average molecular weight of the [OCH₂CH₂]ₙ moiety in the peptide, for example, the number average molecular weight, is 10 kDa.

As the composition according to any one of the above-described embodiments, the X is linked via a sulfur atom of cysteine in the peptide.

As the composition according to any one of the above-described embodiments, the immunoglobulin Fc region is derived from IgG4.

As the composition according to any one of the above-described embodiments, the F has a structure in which two polypeptide chains are linked by a disulfide bond, and are linked only through a nitrogen atom in one of the two chains.

As the composition according to any one of the above-described embodiments, the F includes a monomer of the amino acid sequence of SEQ ID NO: 69.

As the composition according to any one of the above-described embodiments, the F is a homodimer of the monomers of the amino acid sequence of SEQ ID NO: 69.

As the composition according to any one of the above-described embodiments, the F is linked through a nitrogen atom of proline at the N-terminus thereof.

As the composition according to any one of the above-described embodiments, the F, which is an immunoglobulin Fc region, and X are non-glycosylated.

Another aspect for implementing the present invention provides a pharmaceutical composition for preventing or treating liver disease, including a pharmaceutically acceptable excipient and a peptide including any one of the amino acid sequences of SEQ ID NOS: 20, 22, 33, 37, and 38 in a pharmaceutically effective amount.

In one embodiment, the peptide is in the form of a long-acting conjugate, and the long-acting conjugate is represented by Chemical Formula 1 below:

[Chemical Formula 1] X-L-F

with the proviso that X is a peptide including any one of the amino acid sequences of SEQ ID NOS: 20, 22, 33, 37, and 38;
L is a linker containing ethylene glycol repeating units;
F is an immunoglobulin Fc region; and
- represents a covalent bond between X and L, and L and F.

In another embodiment, the liver disease is any one selected from the group consisting of steatosis, liver fibrosis, liver inflammation, liver cirrhosis, liver decompensation, hepatocellular carcinoma, and cholestasis liver disease.

As the composition according to any one of the above-described embodiments, the cholestasis liver disease is any one selected from the group consisting of primary biliary cirrhosis, primary sclerosing cholangitis, and a combination thereof.

As the composition according to any one of the above-described embodiments, the liver disease is caused by non-alcoholic steatohepatitis (NASH) or is accompanied thereby, but is not limited thereto.

Still another aspect for implementing the present invention provides a method for preventing or treating liver disease, including administering the peptide or a conjugate including the same, or a composition including the same to a subject in need thereof.

Yet another aspect for implementing the present invention provides the use of the peptide or a conjugate including the same, or a composition including the same in the preparation of a medicament for preventing or treating liver disease.

### [Mode for Carrying Out the Invention]

Hereinafter, the present invention will be described in more detail. Meanwhile, each of the explanations and exemplary embodiments disclosed herein can be applied to each other explanation and exemplary embodiment. That is, all of the combinations of various factors disclosed herein belong to the scope of the present invention. Moreover, the scope of the present invention should not be limited by the specific disclosure provided hereinbelow.

Additionally, those of ordinary skill in the art may be able to recognize or confirm, using only conventional experimentation, many equivalents to the particular aspects of the invention described herein. Furthermore, it is also intended that these equivalents be included in the present invention.

Throughout the entire specification of the present invention, not only the conventional one-letter and three-letter codes for naturally occurring amino acids, but also those three-letter codes generally allowed for other amino acids, such as α-aminoisobutyric acid (Aib), Sar (*N*-methylglycine), *etc.,* are used. Additionally, the amino acids mentioned herein are abbreviated according to the nomenclature rules of IUPAC-IUB as follows:

| | |
|---|---|
| alanine A | arginine R |
| asparagine N | aspartic acid D |
| cysteine C | glutamic acid E |
| glutamine Q | glycine G |
| histidine H | isoleucine I |
| leucine L | lysine K |
| methionine M | phenylalanine F |
| proline P | serine S |
| threonine T | tryptophan W |
| tyrosine Y | valine V |

In the present specification, the "Aib" may be used interchangeably with "2-aminoisobutyric acid" or "aminoisobutyric acid", and 2-aminoisobutyric acid and aminoisobutyric acid may be used interchangeably with each other.

One aspect for implementing the present invention provides a pharmaceutical composition for preventing or treating liver disease, including a peptide or a conjugate including the same. The peptide is a peptide having activity on a glucagon receptor, and may specifically be a glucagon derivative, but is not limited thereto.

The glucagon derivative according to the present invention includes a peptide having at least one difference in the amino acid sequence compared to native glucagon, a peptide in which the sequence of native glucagon is modified by modifying native glucagon, and a native glucagon mimetic that can activate glucagon receptors like native glucagon.

As a kind of the glucagon derivative peptide, the kind described in International Patent Publication Nos. WO 2016/108586 and WO 2017/003191 may be mentioned, and the specifications of the International Patent Publications are incorporated herein by reference in their entirety. Further, the method for preparing the long-acting conjugate of the glucagon derivative peptide is described in WO 2017/003191, and it is obvious that the entire specification of the International Patent Publication is also incorporated herein by reference.

Such a glucagon derivative may be one having improved physical properties by having an altered pl relative to native glucagon.

Additionally, the glucagon derivative may be glucagon which does not occur naturally.

Meanwhile, the native glucagon may have the following amino acid sequence:

His–Ser–Gln–Gly–Thr–Phe–Thr–Ser–Asp–Tyr–Ser–Lys–Tyr–Leu–Asp–Ser–
Arg–Arg–Ala–Gln–Asp–Phe–Val–Gln–Trp–Leu–Met–Asn–Thr (SEQ ID NO: 1)

As used herein, the term "isoelectric point" or "pi" refers to the pH value at which a molecule such as a polypeptide or peptide has no net charge (0). In the case of a polypeptide with various charged functional groups, the net charge of the total polypeptide is "0" at a point where the pH value is the same as that of the pI. The net charge of the polypeptide at a pH higher than the pl will be negative, while the net charge of the polypeptide at a pH lower than the pl will be positive.

The pl values may be determined on an immobilized pH gradient gel consisting of polyacrylamide, starch, or agarose by isoelectric electrophoresis, or may be estimated, for example, from an amino acid sequence using a pl/MW tool (http://expasy.org/tools/pi_tool.html; Gasteiger *et al.,* 2003) in an ExPASy server.

As used herein, the term "altered pi" refers to a pl which is different from that of native glucagon due to the substitution of a part of the amino acid sequence of native glucagon with an amino acid residue having a negative charge or a positive charge, *i.e*., a reduced or increased pl value.

More specifically, the glucagon derivative may have an altered pl value, not the pl value (6.8) of native glucagon, and even more specifically a pl value of less than 6.8, more specifically 6.7 or less, more specifically 6.5 or less, and additionally a pl value exceeding 6.8, 7 or higher, more specifically 7.5 or higher, but is not limited thereto, and any pl value different from that of native glucagon will belong to the scope of the present invention.

More specifically, the glucagon derivative may have a pl value of 4 to 6.5 and/or 7 to 9.5, specifically 7.5 to 9.5, and more specifically 8.0 to 9.3, but the pl value is not limited thereto.

Specifically, a derivative of native glucagon may be modified by any one method of substitution, addition, deletion, and modification, or a combination thereof in part of the amino acid of native glucagon.

Examples of the glucagon derivatives prepared by a combination of the above methods include a peptide which differs in at least one amino acid residue of the amino acid sequence compared to that of native glucagon and in which the N-terminal amino acid residue is deaminated, having the function of activating a glucagon receptor, *etc.,* but is not limited thereto, and the native glucagon derivatives that are applicable to the present invention can be prepared by a combination of various methods for preparing the derivatives.

Additionally, such modification for the preparation of native glucagon derivatives may include all of the modifications using L-type or D-type amino acids, and/or non-native-type amino acids; and/or a modification of native sequence, for example, modification of a functional group, an intramolecular covalent bonding (*e.g*., a ring formation between side chains), methylation, acylation, ubiquitination, phosphorylation, aminohexanation, biotinylation, *etc.* Additionally, the modification may also include substitutions into non-native compounds.

Additionally, the native glucagon derivatives may also include modifications of all those where one or more amino acids are added to the amino and/or carboxy terminal of native glucagon.

As for the amino acids being substituted or added, not only the 20 amino acids commonly found in human proteins, but also atypical amino acids or those which do not occur naturally can be used. Commercial sources of atypical amino acids may include Sigma-Aldrich, ChemPep Inc., and Genzyme Pharmaceuticals. The peptides including these amino acids and typical peptide sequences may be synthesized and purchased from commercial suppliers, *e.g*., American Peptide Company, Bachem (USA), or Anygen (Korea).

Amino acid derivatives may also be obtained in a similar manner, for example, 4-imidazoacetic acid, *etc.,* may be used.

Since glucagon has a pH of about 7, it is insoluble in a solution having a physiological pH (pH 4 to 8) and tends to precipitate at a neutral pH. In an aqueous solution with a pH of 3 or below, glucagon is dissolved at the initial stage but precipitates within one hour by forming a gel. Since the gelated glucagon mainly consists of β-sheet fibrils, the administration of the thus-precipitated glucagon via an injection needle or intravenous injection will block blood vessels, and thus is not suitable for use as an injection agent. In order to delay the precipitation process, acidic (pH 2 to 4) formulations are commonly used, and by doing so, glucagon can be maintained in a relatively non-aggregated state for a short period of time. However, glucagon can form fibrils very rapidly at a low pH, and thus these acidic formulations must be injected upon preparation.

Accordingly, the glucagon derivatives according to the present invention have extended action profiles by modifying the pl of native glucagon via substitution of amino acid residues having negative charges and positive charges.

In the present invention, the glucagon derivative may be a peptide which includes the amino acid sequence of General Formula 1 below, but is not limited thereto:
Y-X2-QGTF-X7-SDYSKY-X14-D-X16-X17-R-X19-X20-X21-FVQWLMNT-X30 (General Formula 1, SEQ ID NO: 46)
wherein,
X2 is aminoisobutyric acid (Aib);
X7 is threonine (T), valine (V), or cysteine (C);
X14 is leucine (L) or cysteine (C);
X16 is glutamic acid (E) or serine (S);
X17 is lysine (K), arginine (R), or cysteine (C);
X19 is alanine (A) or cysteine (C);
X20 is lysine (K) or glutamine (Q);
X21 is aspartic acid (D) or glutamic acid (E); and
X30 is cysteine (C) or is absent (with the proviso that when the amino acid sequence of General Formula 1 is identical to SEQ ID NO: 1 or SEQ ID NO: 12, it is excluded).

For example, the peptide may be one which includes an amino acid sequence selected from the group consisting of SEQ ID NOS: 20, 22, 23, 27, 33, 35, 37, 38, 40, 41, 42, and 44, and specifically one which consists (essentially) of an amino acid sequence selected from the group consisting of SEQ ID NOS: 20, 22, 23, 27, 33, 35, 37, 38, 40, 41, 42, and 44, but is not limited thereto.

Specifically, the peptide may be one which includes an amino acid sequence selected from the group consisting of SEQ ID NOS: 20, 22, 23, 27, 33, 37, 38, and 44, and specifically one which consists (essentially) of an amino acid sequence selected from the group consisting of SEQ ID NOS: 20, 22, 23, 27, 33, 37, 38, and 44, but is not limited thereto.

In another embodiment, the peptide of the present invention may be one which includes an amino acid sequence selected from the group consisting of SEQ ID NOS: 2 to 45, and specifically one which consists (essentially) of an amino acid sequence selected from the group consisting of SEQ ID NOS: 2 to 45, but is not limited thereto. Additionally, the peptide of the present invention may be one which includes an amino acid sequence selected from the group consisting of SEQ ID NOS: 2 to 11, and 13 to 45, and specifically one which consists (essentially) of an amino acid sequence selected from the group consisting of SEQ ID NOS: 2 to 11, and 13 to 45, but is not limited thereto.

Although it is described as "a peptide consisting of a particular SEQ ID NO" in the present invention, such expression does not exclude a mutation in the peptide that can occur by a meaningless sequence addition upstream or downstream of the amino acid sequence of the corresponding SEQ ID NO, or a naturally occurring mutation therein, or a silent mutation therein, as long as the peptide having such mutation has an activity the same as or corresponding to that of the peptide which consists of an amino acid sequence of the corresponding SEQ ID NO. Even when the sequence addition or a mutation is present, it obviously belongs to the scope of the present invention.

Those described above may be also applied to other specific embodiments or aspects of the present invention, but are not limited thereto.

In the present invention, the peptide may have a sequence identity of at least 60% or more, 65% or more, 70% or more, 75% or more, 80% or more, 85% or more, 90% or more, 91% or more, 92% or more, 93% or more, 94% or more, or 95% or more to the native glucagon, but is not particularly limited thereto, and those skilled in the art can easily identify the sequence identity by sequence comparison of the peptide and native glucagon.

As used herein, the term "homology" is intended to indicate the degree of similarity to the amino acid sequence of a wild-type protein or a base sequence encoding the same and includes sequences having an identity of the above percentage or higher with the amino acid sequence or base sequence of the present invention. The homology may be determined by comparing the two given sequences by the naked eye, but may be determined using a bioinformatic algorithm, which enables the analysis of a homology by arranging the subject sequences for comparison. The homology between the two given amino acid sequences may be indicated as a percentage. The useful automated algorithm is available for use in GAP, BESTFIT, FASTA, and TFASTA computer software modules of Wisconsin Genetics Software Package (Genetics Computer Group, Madison, W, USA). The arrangement algorithm automated in the above modules includes sequence arrangement algorithm by Needleman & Wunsch, Pearson & Lipman, and Smith & Waterman. Other useful algorithms on sequence arrangement and homology determination are automated in software including FASTP, BLAST, BLAST2, PSIBLAST, and CLUSTAL W.

Additionally, in the present invention, the peptide may be a peptide or glucagon derivative exhibiting activity on a glucagon receptor, but is not limited thereto.

Although not particularly limited thereto, the peptide exhibiting a significant level of activity on the glucagon receptor may exhibit *in vitro* activity on the glucagon receptor of about 0.001 % or more, about 0.01 % or more, about 0.1% or more, about 1% or more, about 2% or more, about 3% or more, about 4% or more, about 5% or more, about 6% or more, about 7% or more, about 8% or more, about 9% or more, about 10% or more, about 20% or more, about 30% or more, about 40% or more, about 50% or more, about 60% or more, about 70% or more, about 80% or more, about 90% or more, about 100% or more relative to that of a native ligand (native glucagon), but ranges exhibiting a significant level of activity are included without limitation. A method for measuring the *in vitro* activity of these peptides may refer to Example 4 of the present specification, but is not particularly limited thereto.

As used herein, the term "about" refers to a range which includes all of ±0.5, ±0.4, ±0.3, ±0.2, ±0.1, *etc.* and includes all of the values that are equivalent or similar to those following the values, but the range is not limited thereto.

Although not particularly limited thereto, the peptide may be one which does not occur naturally.

The above-described glucagon derivative may include an intramolecular bridge, *e.g.*, a covalent bridge or a non-covalent bridge, and specifically may be in a form including a ring. For example, the glucagon derivative may be in a form where a ring is formed between the 16^{th} and 20^{th} amino acids of the glucagon derivative, but is not particularly limited thereto.

Non-limiting examples of the ring may include a lactam bridge (or lactam ring).

Additionally, the glucagon derivative includes all of those which are modified to include an amino acid capable of forming a ring at the desired site so as to include a ring.

The ring may be formed between side chains of amino acids within the glucagon derivative (*e.g*., in the form of a ring formation between a side chain of lysine and a side chain of a glutamic acid), but is not particularly limited thereto.

For example, the peptide including the amino acid sequence of General Formula 1 may be one in which each amino acid in each amino acid pair among the amino acid pairs of X10 and X14, X12 and X16, X16 and X20, X17 and X21, X20 and X24, and X24 and X28 in General Formula 1 may be substituted with glutamic acid or lysine, respectively, but is not limited thereto. In the Xₙ (n is an integer), n refers to the position of the amino acid from the N-terminus of an amino acid sequence provided.

Additionally, the peptide including the amino acid sequence of General Formula 1 may be one in which each amino acid in each amino acid pair of X12 and X16 or the amino acid pair of X16 and X20 or the amino acid pair of X17 and X21 is respectively substituted with glutamic acid or lysine, which is capable of forming a ring, but is not limited thereto.

Additionally, in General Formula 1, the peptide may be one in which a ring (*e.g*., a lactam ring) is formed between each amino acid in each amino acid pair of at least one amino acid pair among the amino acid pairs of X10 and X14, X12 and X16, X16 and X20, X17 and X21, X20 and X24, and X24 and X28, but is not limited thereto.

Additionally, in General Formula 1, X16 may be glutamic acid, X20 may be lysine, and the side chains of X16 and X20 may form a lactam ring, but they are not limited thereto.

Additionally, the peptide according to the present invention may be in the form where the N-terminus and/or C-terminus is not modified, however, those variants, where the amino terminus and/or carboxy terminus, *etc.* of the peptide is chemically modified or protected by organic groups, or amino acids added to the end of the peptide for its protection from proteases *in vivo* while increasing its stability, may also be included in the scope of the peptides of the present invention. In a case where the C-terminus is not modified, the end of the peptide according to the present invention may have a carboxyl group, but is not particularly limited thereto.

Additionally, in the case of a chemically synthesized peptide, its N- and C-termini are electrically charged, and thus, in order to remove such electrical charge, the N-termini of the peptide may be acetylated and/or the C-termini of the peptide may be amidated, but the peptide is not particularly limited thereto.

Unless specified otherwise in the present invention, the description in the detailed description or claims with respect to "the peptide" according to the present invention or a "conjugate", in which such a peptide is covalently linked to a biocompatible material, may be applied to the forms, which include not only include the corresponding peptide or conjugate but also the salts of the corresponding peptide or conjugate (*e.g.*, pharmaceutically acceptable salts thereof), or solvates thereof. Accordingly, even in a case where a "peptide" or "conjugate" is described in the present invention, the description may also be equally applied to a particular salt thereof, a particular solvate thereof, and a particular solvate of the particular salt thereof. These salts may be, for example, in a form where any pharmaceutically acceptable salts are used. The kind of the salt is not particularly limited. However, the salt is preferably one that is safe and effective to a subject, *e.g.*, a mammal, but is not particularly limited thereto.

The term "pharmaceutically acceptable" refers to a material which can be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.;* alkali earth metals such as magnesium; ammonium; *etc.*

Additionally, as used herein, the term "solvate" refers to a complex formed between the peptide, conjugate, or a salt thereof according to the present invention and a solvent molecule.

The C-terminus of the peptide according to the present invention may be an amidated peptide or a peptide having a free carboxyl group (-COOH), or may include a peptide having an unmodified C-terminus, but is not limited thereto.

In one embodiment, the peptide may be amidated at the C-terminus, but is not limited thereto.

In one embodiment, the peptide may be non-glycosylated, but is not limited thereto.

The peptide of the present invention can be synthesized through a solid phase synthesis method, can be produced by a recombinant method, and can be produced by commercially, but is not limited thereto.

Additionally, the peptide of the present invention may be synthesized by a method well known in the art, according to its length, *e.g*., by an automatic peptide synthesizer, and may be produced by genetic engineering technology.

Specifically, the peptide of the present invention may be prepared by a standard synthesis method, a recombinant expression system, or any other method known in the art. Accordingly, the glucagon derivative of the present invention may be synthesized by various methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture; or
(c) a method of performing an *in vitro* cell-free expression of a nucleic acid construct encoding a peptide and recovering the expression product therefrom; or
   a method of obtaining peptide fragments by any combination of the methods (a), (b), and (c), obtaining the peptide by linking the peptide fragments, and then recovering the peptide.

In a more specific example, a desired glucagon derivative may be produced by genetic manipulation, which includes preparing a fusion gene encoding a fusion protein, including a fusion partner and a glucagon derivative, transforming the resultant into a host cell, expressing in the form of a fusion protein, and cleaving the glucagon derivative from the fusion protein using a protease or a compound, followed by separation. For this purpose, for example, an amino acid residue-encoding DNA sequence that can be cleaved by a protease such as Factor Xa or enterokinase, CNBr, or a compound such as hydroxylamine, may be inserted between the fusion partner and a polynucleotide encoding a glucagon derivative.

In a more specific embodiment, the peptide according to the present invention, or the glucagon derivative may be in the form of a long-acting conjugate in which a biocompatible material moiety capable of increasing *in vivo* half-life thereof is linked to the peptide or the glucagon derivative, but is not limited thereto. The biocompatible material moiety may be interchangeably used with a carrier.

As used herein, the term "long-acting conjugate", being in the form where a biocompatible material moiety or carrier is linked to a physiologically active material (*e.g*., a glucagon derivative), specifically includes a peptide moiety and a biocompatible material moiety which is covalently linked to the peptide moiety, and the peptide moiety may be represented by the amino acid sequence of General Formula 1 above, or may be a sequence identical to SEQ ID NOS: 2 to 11, and 13 to 45, or a sequence including the same. In the long-acting conjugate, the biocompatible material moiety or carrier may be one that is covalently linked to the physiologically active material, but is not particularly limited thereto.

In the present invention, the conjugate of the peptide may exhibit an enhanced efficacy of duration compared to that of a peptide to which a carrier is not linked, and in the present invention, the conjugate is referred to as a "long-acting conjugate".

Meanwhile, the conjugate may be one which does not occur naturally.

In a specific embodiment of the present invention, the long-acting conjugate may refer to one in which an immunoglobulin Fc region and a glucagon derivative are linked to each other. Specifically, the conjugate may be one in which an immunoglobulin Fc region is covalently linked to a glucagon derivative through a linker, but is not particularly limited thereto.

In one embodiment, the immunoglobulin Fc region and X may not be glycosylated, but is not limited thereto.

In one embodiment of the present invention, the long-acting conjugate may be a conjugate represented by Chemical Formula 1 below, but is not limited thereto:

[Chemical Formula 1] X-L-F

with the proviso that X is the peptide above;
L is a linker containing ethylene glycol repeating units;
F is an immunoglobulin Fc region; and
- represents a covalent bond between X and L, and L and F.

The X of the long-acting conjugate of Chemical Formula 1 may be the glucagon derivative described above, but is not limited thereto.

Specifically, the X may be a peptide including the amino acid sequence of General Formula 1, alternatively, the X may be a peptide including any one of the amino acid sequences of SEQ ID NOS: 2 to 45, or a peptide including any one of the amino acid sequences of SEQ ID NOS: 2 to 11 and 13 to 45, or the X may be a peptide including an amino acid sequence selected from the group consisting of SEQ ID NOS: 20, 22, 23, 27, 33, 35, 37, 38, 40, 41, 42, and 44, but is not limited thereto.

As used herein, the term "long-acting conjugate of Chemical Formula 1" being in the form where a glucagon derivative and an immunoglobulin Fc region are linked to each other via a linker, may exhibit an enhanced efficacy of duration compared to a glucagon derivative to which an immunoglobulin Fc region is not linked.

In the long-acting conjugate, F is a substance capable of increasing the half-life of X, *i.e.,* a glucagon derivative, and corresponds to one constitution of the moiety constituting the conjugate of the present invention.

In the long-acting conjugate of Chemical Formula 1, the linkage between X, the peptide, which is the glucagon derivative, and the immunoglobulin Fc region may be achieved by a physical or chemical bond, a non-covalent or covalent bond, and specifically a covalent bond, but is not limited thereto.

Additionally, the method of linking X (*i.e*., the glucagon derivative) and the immunoglobulin Fc region of the long-acting conjugate of Chemical Formula 1 is not particularly limited, but the glucagon derivative and the immunoglobulin Fc region may be linked to each other through a linker.

In Chemical Formula 1 above, X and F may be linked to each other through L by a covalent bond.

More specifically, X and L, and L and F may be linked to each other by a covalent bond, and in particular, the conjugate may be a conjugate in which X, L, and F are each linked by a covalent bond in the order of Chemical Formula 1.

Additionally, the X may be directly linked to F (*i.e*., a is 0 in Chemical Formula 1 above) or may be linked through a linker (L).

In a specific example, the F may be an immunoglobulin Fc region, and more specifically, the immunoglobulin Fc region may be derived from IgG, but is not particularly limited thereto.

As used herein, the term "immunoglobulin Fc region" refers to a region including the heavy chain constant region 2 (CH2) and/or the heavy chain constant region 3 (CH3), excluding the heavy chain and light chain variable regions of an immunoglobulin. The immunoglobulin Fc region may be one constitution constituting a moiety of the long-acting conjugate of the present invention.

The immunoglobulin Fc region may be used interchangeably with "immunoglobulin Fc fragment".

The Fc region in the present specification encompasses not only a native sequence obtained from papain digestion of an immunoglobulin, but also derivatives thereof, for example, variants, in which one or more amino acid residues in the native sequence are converted by deletion, insertion, non-conservative or conservative substitution, or a combination thereof, and thus the sequence become different from the native sequence, *etc.* The above derivatives, substituents, and variants are required to retain FcRn-binding ability. In the present invention, F may be a human immunoglobulin region, but is not limited thereto. The F may have a structure in which two polypeptide chains are linked by a disulfide bond, or a structure in which two polypeptide chains are linked through a nitrogen atom in only one of the two chains, but is not limited thereto. The linkage through the nitrogen atom may be linked to the epsilon amino atom or the N-terminal amino group of lysine via reductive amination.

The reductive amination reaction refers to a reaction in which an amine group or amino group of a reactant reacts with an aldehyde of another reactant (*i.e*., a functional group capable of reductive amination) to produce an amine, and an amine bond is formed by a reduction reaction thereafter. The reductive amination reaction is a reaction of organic synthesis widely known in the art.

In one embodiment, the F may be linked through the nitrogen atom of the N-terminal proline thereof, but is not limited thereto.

Such an immunoglobulin Fc region may include a hinge region in the heavy chain constant region, but is not limited thereto.

In the present invention, the immunoglobulin Fc region may include a specific hinge sequence in the N-terminus.

As used herein, the term "hinge sequence" refers to a region which is located in the heavy chain and forms a dimer of immunoglobulin Fc regions through an inter-disulfide bond.

In the present invention, the hinge sequence may be modified such that a part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 49).

The hinge sequence may be one in which the 8^{th} or 11^{th} cysteine residue in the hinge sequence of SEQ ID NO: 49 is deleted and thus only one cysteine residue is included in the sequence. The hinge sequence of the present invention may consist of 3 to 12 amino acids, including only one cysteine residue, but the hinge sequence is not limited thereto. More specifically, the hinge sequence of the present invention may have the following sequences:
Glu-Ser-Lys-Tyr-Gly-Pro-Pro-Pro-Ser-Cys-Pro (SEQ ID NO: 50), Glu-Ser-Lys-Tyr-Gly-Pro-Pro-Cys-Pro-Ser-Pro (SEQ ID NO: 51), Glu-Ser-Lys-Tyr-Gly-Pro-Pro-Cys-Pro-Ser (SEQ ID NO: 52), Glu-Ser-Lys-Tyr-Gly-Pro-Pro-Cys-Pro-Pro (SEQ ID NO: 53), Lys-Tyr-Gly-Pro-Pro-Cys-Pro-Ser (SEQ ID NO: 54), Glu-Ser-Lys-Tyr-Gly-Pro-Pro-Cys (SEQ ID NO: 55), Glu-Lys-Tyr-Gly-Pro-Pro-Cys (SEQ ID NO: 56), Glu-Ser-Pro-Ser-Cys-Pro (SEQ ID NO: 57), Glu-Pro-Ser-Cys-Pro (SEQ ID NO: 58), Pro-Ser-Cys-Pro (SEQ ID NO: 59), Glu-Ser-Lys-Tyr-Gly-Pro-Pro-Ser-Cys-Pro (SEQ ID NO: 60), Lys-Tyr-Gly-Pro-Pro-Pro-Ser-Cys-Pro (SEQ ID NO: 61), Glu-Ser-Lys-Tyr-Gly-Pro-Ser-Cys-Pro (SEQ ID NO: 62), Glu-Ser-Lys-Tyr-Gly-Pro-Pro-Cys (SEQ ID NO: 63), Lys-Tyr-Gly-Pro-Pro-Cys-Pro (SEQ ID NO: 64), Glu-Ser-Lys-Pro-Ser-Cys-Pro (SEQ ID NO: 65), Glu-Ser-Pro-Ser-Cys-Pro (SEQ ID NO: 66), Glu-Pro-Ser-Cys (SEQ ID NO: 67), Ser-Cys-Pro (SEQ ID NO: 68).

More specifically, the hinge sequence may include the amino acid sequence of SEQ ID NO: 59 (Pro-Ser-Cys-Pro) or SEQ ID NO: 68 (Ser-Cys-Pro), but is not limited thereto.

The immunoglobulin Fc region of the present invention may be in a form in which two molecules of the immunoglobulin Fc chain form a dimer due to the presence of a hinge sequence therein, and in addition, the long-acting conjugate of Chemical Formula 1 of the present invention may be in a form in which one end of the linker is linked to one chain of the dimeric immunoglobulin Fc regions, but the immunoglobulin Fc region and the conjugate are not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the most terminal end of the amino terminus. The immunoglobulin Fc region of the present invention may include a hinge sequence in the N-terminus, but is not limited thereto.

Additionally, the immunoglobulin Fc region of the present invention may be an extended Fc region, which includes all or part of the heavy chain constant region 1 (CH1) and/or light chain constant region 1 (CL1) excluding the heavy chain and light chain variable regions of an immunoglobulin, as long as it has substantially the same or an improved effect compared to its native type. Additionally, the immunoglobulin Fc region of the present invention may be a region in which some fairly long amino acid sequences corresponding to CH2 and/or CH3 are removed.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain, and a CH4 domain and an immunoglobulin hinge region (or part of the hinge region); and 6) a dimer of each domain of the heavy chain constant region and a light chain constant region, but the immunoglobulin Fc region is not limited thereto.

In the present invention, the immunoglobulin Fc region may be in the form of a dimer or a multimer, composed of single-chain immunoglobulins consisting of domains of the same origin, but is not limited thereto.

Additionally, in one embodiment, the immunoglobulin Fc region may have a dimeric form, and one molecule of X may be covalently linked to one Fc region in a dimeric form, in particular, the immunoglobulin Fc and X may be covalently linked to each other through a non-peptidyl polymer. Meanwhile, it is also possible that two molecules of X are symmetrically conjugated to one Fc region in a dimeric form. In particular, the immunoglobulin Fc and X may be linked to each other through a non-peptidyl polymer, but is not limited to the embodiments described above.

In addition, the immunoglobulin Fc region of the present invention includes natural amino acid sequences as well as sequence derivatives thereof. The amino acid sequence derivative means that the sequence of the amino acid is different from that of its natural amino acid due to deletion, insertion, non-conservative or conservative substitution, or a combination thereof in one or more amino acid residues in the sequence of the natural amino acid.

For example, amino acid residues at positions 214 to 238, 297 to 299, 318 to 322, or 327 to 331 in IgG Fc, which are known to be important for linkage, may be used as the sites suitable for modification.

Additionally, various types of derivatives are possible, for example, one where the site capable of forming a disulfide bond is removed; one where several N-terminal amino acids from native Fc are removed; one where a methionine residue is added to the N-terminus of native Fc, *etc.* Additionally, complement binding sites (*e.g*., C1q binding sites) or antibody-dependent cell-mediated cytotoxicity (ADCC) sites may be removed to remove the effector function. The techniques for preparing the sequence derivatives of these immunoglobulin Fc regions are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

The Fc derivatives described above may be those which exhibit the same biological activity as the Fc region of the present invention and have an increased structural stability of the Fc region against heat, pH, *etc.*

Additionally, such an Fc region may be obtained from a native type isolated from humans or animals (*e.g*., cows, goats, pigs, mice, rabbits, hamsters, rats, guinea pigs, *etc.)* or may be recombinants or derivatives thereof obtained from transformed animal cells or microorganisms. In particular, the Fc region may be obtained from a native immunoglobulin by isolating a whole immunoglobulin from a living human or animal and treating the isolated immunoglobulin with protease. When the whole immunoglobulin is treated with papain, it is cleaved into Fab and Fc regions, whereas when treated with pepsin, it is cleaved into pF'c and F(ab)₂ regions. Fc or pF'c can be isolated using size exclusion chromatography, *etc.* In a more specific embodiment, the Fc region may be a recombinant immunoglobulin Fc region where a human-derived Fc region is obtained from a microorganism.

Additionally, the immunoglobulin Fc region may be in the form of native glycans, increased or decreased glycans compared to the native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc region where the glycans are removed from the Fc shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc region in a deglycosylated or aglycosylated form may be more suitable to meet the original object of the present invention as a drug carrier.

As used herein, the term "deglycosylation" refers to an Fc region in which glycans are removed with an enzyme, and the term "aglycosylation" refers to a non-glycosylated Fc region produced in prokaryotes, more specifically *E*. *coli.*

Meanwhile, the immunoglobulin Fc region may be derived from humans or animals (*e.g*., cows, goats, pigs, mice, rabbits, hamsters, rats, guinea pigs, *etc*.), and in a more specific embodiment, it may be derived from humans.

Additionally, the immunoglobulin Fc region may be derived from IgG, IgA, IgD, IgE, IgM, or a combination or hybrid thereof. In a more specific embodiment, the immunoglobulin Fc region may be derived from IgG or IgM, which are among the most abundant proteins in human blood, and in an even more specific embodiment, it may be derived from IgG, which is known to enhance the half-lives of ligand-binding proteins. In an even yet more specific embodiment, the immunoglobulin Fc region may be an IgG4 Fc region, and in the most specific embodiment, it may be an aglycosylated Fc region derived from a human IgG4, but is not limited thereto.

Additionally, in a specific embodiment, the immunoglobulin Fc region, being a human IgG4 Fc region, may be in the form of a homodimer in which two monomers are linked through a disulfide bond (an inter-chain form) between cysteines, which are the 3^{rd} amino acid of each monomer. In particular, each monomer of the homodimer independently has/or can have an internal disulfide bond between the cysteines at positions 35 and 95; and an internal disulfide bond between the cysteines at positions 141 and 199 (*i.e*., two internal disulfide bonds (an intra-chain form)). With respect to the number of amino acids, each monomer may consist of 221 amino acids, and the amino acids forming the homodimer may consist of a total of 442 amino acids, but the number of amino acids is not limited thereto. Specifically, the immunoglobulin Fc region may be one in which two monomers having the amino acid sequence of SEQ ID NO: 69 (consisting of 221 amino acids) form a homodimer through a disulfide bond between cysteines, which are the 3^{rd} amino acid of each monomer, and in which the monomers of the homodimer independently form an internal disulfide bond between the cysteines at positions 35 and 95 and an internal disulfide bond between the cysteines at positions 141 and 199, but the immunoglobulin Fc region is not limited thereto.

The F in Chemical Formula 1 may include a monomer of the amino acid sequence of SEQ ID NO: 69, and the F may be a homodimer of the monomers of the amino acid sequence of SEQ ID NO: 69, but is not limited thereto.

In one example, the immunoglobulin Fc region may be a homodimer including the amino acid sequence of SEQ ID NO: 70 (consisting of 442 amino acids), but is not limited thereto.

Meanwhile, as used herein, the term "combination" means that polypeptides encoding single-chain immunoglobulin Fc regions of the same origin are linked to a single-chain polypeptide of a different origin to form a dimer or multimer. That is, it is possible to prepare a dimer or multimer from two or more regions selected from the group consisting of Fc regions of IgG Fc, IgA Fc, IgM Fc, IgD Fc, and IgE Fc.

Meanwhile, the L may be a non-peptidyl linker, for example, a linker containing an ethylene glycol repeating unit.

In the present invention, the term "non-peptidyl linker" includes a biocompatible polymer in which two or more repeating units are linked. The repeating units are linked to each other through any covalent bond which is not a peptide bond. The non-peptidyl linker may be one constitution that constitutes the moiety of the conjugate of the present invention, and it corresponds to L in Chemical Formula 1 above. As the non-peptidyl linker that can be used in the present invention, any polymer which has a resistance to proteases *in vivo* can be used without limitation. In the present invention, the non-peptidyl linker can be used interchangeably with a non-peptidyl polymer.

Although not particularly limited, the non-peptide linker may be a linker containing an ethylene glycol repeating unit (*e.g*., polyethylene glycol), and additionally, those derivatives which are already known in the art and the derivatives that can easily be prepared at the technological level of those skilled in the art are included in the scope of the present invention.

The repeating unit of the non-peptide linker may be an ethylene glycol repeating unit, and specifically, the non-peptide linker may be one which includes a functional group used for the preparation of the conjugate at an end while including an ethylene glycol repeating unit. The long-acting conjugate according to the present invention may be in the form in which X and F are linked through the functional group, but the long-acting conjugate is not limited thereto. In the present invention, the non-peptide linker may include two, or three or more functional groups, and each functional group may be the same as or different from each other, but the non-peptide linker is not limited thereto.

Specifically, the linker may be polyethylene glycol (PEG) represented by Chemical Formula 2 below, but the linker is not limited thereto: 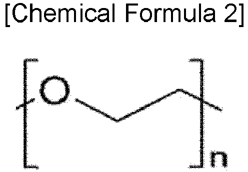 wherein n is 10 to 2,400, n is 10 to 480, or n is 50 to 250, but the range of n is not limited thereto.

In the long-acting conjugate above, the PEG moiety may include not only the -(CH₂CH₂O)ₙ- structure, but also an oxygen atom interposed between a linking element and the -(CH₂CH₂O)ₙ- structure, but the PEG moiety is not limited thereto.

In one embodiment, the ethylene glycol repeating unit may be represented by, for example, [OCH₂CH₂]ₙ, and the value of n is a natural number and the average molecular weight of the [OCH₂CH₂]ₙ region in the peptide conjugate, for example, the number average molecular weight, may be determined to be greater than 0 kDa to about 100 kDa, but is not limited thereto. In another example, the value of n is a natural number, and the average molecular weight of the [OCH₂CH₂]ₙ region in the peptide conjugate, for example, the number average molecular weight, may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, or about 10 kDa, but is not limited thereto.

Additionally, in a specific embodiment, the conjugate may have a structure in which an immunoglobulin region (F) is linked to a peptide (X) including the amino acid sequence of General Formula 1 by a covalent bond through a linker containing an ethylene glycol repeating unit, but the structure of the conjugate is not limited thereto.

The polyethylene glycol is a general term including all of the forms of homopolymers of ethylene glycol, PEG copolymers, or monomethyl-substituted PEG polymers (mPEG), but the polyethylene glycol is not particularly limited thereto.

The molecular weight of the non-peptidyl polymer may be in the range of 0 kDa to about 100 kDa, specifically 1 kDa to 20 kDa, or 1 kDa to 10 kDa, but the molecular weight of the non-peptidyl polymer is not limited thereto. Additionally, the non-peptidyl linker of the present invention, which is linked to the polypeptide corresponding to the F, may include not only a single kind of a polymer but also a combination of different kinds of polymers.

In one embodiment, both ends of the linker may be bound to a thiol group, an amino group, or a hydroxyl group of the immunoglobulin Fc region, and may be bound to a thiol group, an amino group, an azide group, or a hydroxyl group of the peptide (X).

Specifically, the linker may include a reactive group capable of binding to each of the immunoglobulin Fc region and the peptide (X) at both ends. Specifically, the linker may include a reactive group that can bind to a thiol group of cysteine; an amino group located at the N-terminus, lysine, arginine, glutamine, and/or histidine; and/or a hydroxyl group located at the C-terminus of the immunoglobulin Fc region, and that can bind to a thiol group of cysteine; an amino group of lysine, arginine, glutamine, and/or histidine; an azide group of azido-lysine; and/or a hydroxyl group of the peptide (X), but the reactive groups are not limited thereto.

More specifically, the reactive group of the linker may be one or more selected from the group consisting of an aldehyde group, a maleimide group, and a succinimide derivative, but is not limited thereto.

In the above, as an example of the aldehyde group, a propionaldehyde group or butyraldehyde group may be used, but the aldehyde group is not limited thereto.

In the above, as a succinimide derivative, succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate may be used, but the succinimide derivative is not limited thereto.

The linker may be linked to F, the immunoglobulin Fc region, and X, the peptide (glucagon derivative), through the reactive groups to be converted to a linker moiety.

Additionally, the final product produced through reductive amination by an aldehyde bond is much more stable than that linked by an amide bond. The aldehyde reactive group selectively reacts at the N-terminus at a low pH, while it can form a covalent bond with a lysine residue at a high pH (*e.g*., pH 9.0), but is not limited thereto.

The terminal reactive groups of the linker of the present invention may be the same as or different from each other. The linker may have an aldehyde reactive group at both ends. Alternatively, the linker may have an aldehyde group and a maleimide group at each end, or may have an aldehyde group and a succinimide reactive group at each end, but is not limited thereto.

For example, the linker may have a maleimide group at one end and an aldehyde group, a propionaldehyde group, or a butyraldehyde group at the other end. As another example, the linker may have a succinimidyl group at one end and a propionaldehyde group or a butyraldehyde group at the other end.

When a polyethylene glycol having a hydroxy reactive group at propionaldehyde end is used as a linker, the conjugate of the present invention may be prepared by activating the hydroxy group to various reactive groups by known chemical reactions or by using a commercially available polyethylene glycol having a modified reactive group.

In a specific embodiment, the reactive group of the linker may be linked to a cysteine residue of the peptide, more specifically to the -SH group of cysteine, but the linker is not limited thereto.

When maleimide-PEG-aldehyde is used, the maleimide group may be linked to the -SH group of the peptide by a thioether bond, and the aldehyde group may be linked to the -NH₂ group of the immunoglobulin Fc through reductive alkylation, but is not limited thereto, and this is merely an embodiment.

Through the reductive alkylation, a structure such as -PEG-O-CH₂CH₂CH₂NH-immunoglobulin Fc may be formed by linking an amino group at the N-terminus of an immunoglobulin Fc region to an oxygen atom located at one end of the PEG through a linker reactive group having a structure of -CH₂CH₂CH₂-; and a structure, in which one end of the PEG is linked to a sulfur atom located at the cysteine of the peptide through a thioether bond, may be formed. The thioether bond described above may include the following structure:

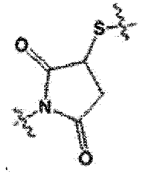

However, the linker is not particularly limited to the above embodiment, and it is merely an embodiment.

Additionally, in the above conjugate, the reactive group of the linker may be linked to -NH₂ located at the N-terminus of the immunoglobulin Fc region, but this is merely an embodiment.

In addition, in the conjugate, the peptide according to the present invention may be linked to a linker having a reactive group through the C-terminus, but this is merely an embodiment.

As used herein, the term "C-terminus" refers to a carboxy terminus of a peptide, and refers to a position capable of binding with the linker for the purpose of the present invention. For example, the C-terminus may include all of the amino acid residue at the most terminal end of the C-terminus and amino acid residues near the C-terminus, and specifically may include the 1^{st} to 20^{th} amino acid residues from the most terminal end, although the C-terminus is not limited thereto.

In one embodiment, the conjugate of Chemical Formula 1 may have a structure of Chemical Formula 3 below:

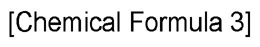

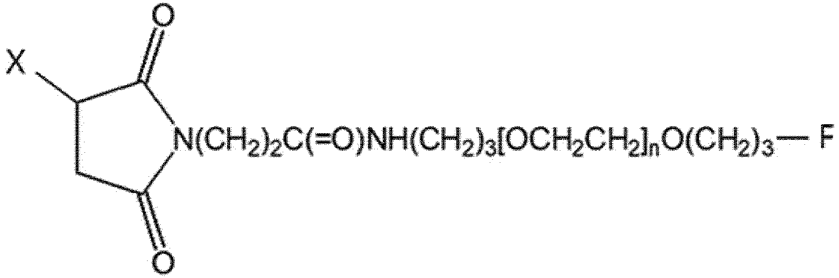

In Chemical Formula 3, X is the peptide (glucagon derivative) described above;
F is a human immunoglobulin Fc region; and
n may be a natural number. In particular, the description of n is the same as described above.

In one embodiment, the long-acting conjugate of Chemical Formula 3 has a structure in which the peptide X and the immunoglobulin Fc region F are covalently linked through an ethylene glycol repeating unit, wherein each X may be linked to a succinimide ring of Chemical Formula 3, and F may be linked to an oxypropylene group of Chemical Formula 3.

In Chemical Formula 3 above, the value of n may be determined such that the average molecular weight of the [OCH₂CH₂]ₙ region in the peptide conjugate, for example, the number average molecular weight, is 1 kDa to 100 kDa, or 1 kDa to 20 kDa, or 10 kDa, but not limited thereto.

In one embodiment, the moiety at which X is linked to the succinimide ring of Chemical Formula 3 may be a sulfur atom of the C-terminal cysteine of X.

The moiety linked to the oxypropylene group in F is not specifically limited. In one embodiment of the present invention, the moiety of F linked to the oxypropylene group may be an N-terminal nitrogen or a nitrogen atom of a residue in F (*e.g*., epsilon nitrogen of lysine). In one specific embodiment of the present invention, the moiety where F is linked to the oxypropylene group may be the N-terminal proline of F, but is not limited thereto.

In addition, the above-described conjugate may have an enhanced efficacy of duration compared to native glucagon or X which is not modified with F, and such a conjugate is not only in the above-described form, but also in the form encapsulated in biodegradable nanoparticles.

The peptide according to the present invention or a conjugate thereof may have a preventive or therapeutic use for liver disease. Specifically, the peptide according to the present invention or a conjugate thereof, or a composition including the same may have a preventive or therapeutic use for liver disease by exhibiting an effect of inhibiting and improving inflammation and/or fibrosis in liver tissue, but is limited thereto. In addition, the peptide according to the present invention or a conjugate thereof, or a composition including the same may reduce fat content in liver tissue to improve excessive fat deposition that causes not only steatosis but also various liver diseases, but is not limited thereto.

As used herein, the term "liver disease" refers to a disease occurring in the liver, and it may be any one selected from the group consisting of steatosis, liver fibrosis, liver inflammation, liver cirrhosis, liver decompensation, hepatocellular carcinoma, and cholestasis liver disease, and specifically, the cholestasis liver disease may be primary biliary cirrhosis, primary sclerosing cholangitis, and a combination thereof, and as long as an abnormality occurs in the tissues and functions of the liver, the liver disease is not limited to those described above.

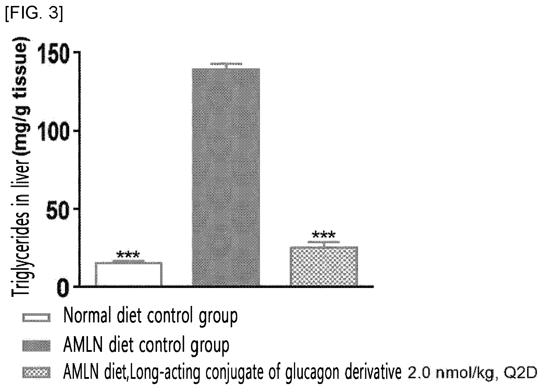

In the Examples of the present invention, since the effect of reducing triglycerides in liver tissue was confirmed in a non-alcoholic fatty liver mouse model induced by AMLN (amylin) diet (FIG. 3), the glucagon derivative according to the present invention, a conjugate thereof, or a composition including the same has preventive and therapeutic effects on metabolic liver diseases including steatosis by reducing fat in liver tissue. Moreover, fat deposition in liver tissue induces inflammation, leading to the progression of other liver diseases.

The liver disease, for which the peptide according to the present invention or a conjugate thereof has a therapeutic effect, may be a metabolic liver disease, but is not limited thereto. The metabolic liver disease is a disease caused by an abnormal chemical reaction of the body that interferes with the body's metabolism, and it includes steatosis, fatty liver, steatohepatitis, *etc.*

The composition according to the present invention may be one which exhibits a preventive or therapeutic effect on metabolic liver disease by reducing triglyceride in liver tissue and alleviating inflammation when administered, but is not limited thereto. The metabolic liver disease may or may not be accompanied by inflammation, and examples of liver diseases that can be treated with the composition according to the present invention include steatosis, non-alcoholic fatty liver, and non-alcoholic steatohepatitis, but the liver diseases are not limited thereto.

The "non-alcoholic fatty liver disease (NAFLD)", which is a representative example of metabolic liver disease, refers to the case where this is accompanied by fatty liver even though the subject has no history of alcohol intake or is not related to alcohol intake. Fatty liver refers to the occurrence of a phenomenon in which triglycerides appear to be abnormally deposited in liver cells, unlike normal cases. About 5% of the normal liver is composed of adipose tissue, and although triglycerides, fatty acids, phospholipids, cholesterol, and cholesterol esters are the main components of fat, once fatty liver occurs, most of the components are replaced with triglycerides, and when the amount of triglycerides is 5% or higher relative to the liver weight, it is diagnosed as fatty liver. Fatty liver is caused by a disorder of fat metabolism in liver cells or a defect in the process of transporting excessive fat, *etc.,* and it is mainly caused by a disorder of fat metabolism in the liver. Most of the fat accumulated in the fatty liver may be triglycerides.

The non-alcoholic fatty liver disease refers to a group of diseases which includes simple steatosis with only excessive accumulation of fat in liver cells, non-alcoholic fatty liver, non-alcoholic steatohepatitis (NASH) accompanied by hepatocellular necrosis, inflammation and fibrosis, *etc.,* but the non-alcoholic fatty liver disease is not limited thereto as long as the disease can be treated with the composition according to the present invention. The non-alcoholic fatty liver disease according to the present invention may be one which is accompanied by non-alcoholic steatohepatitis, but the non-alcoholic fatty liver disease is not limited thereto.

In many cases, liver inflammation may be caused by fat deposition, viruses, alcohol, drugs, immune disorders, metabolic diseases, *etc.,* and liver inflammation is known to develop into diseases such as liver fibrosis, cirrhosis, liver decompensation, liver cancer, *etc.* according to the progression and chronicity of liver inflammation.

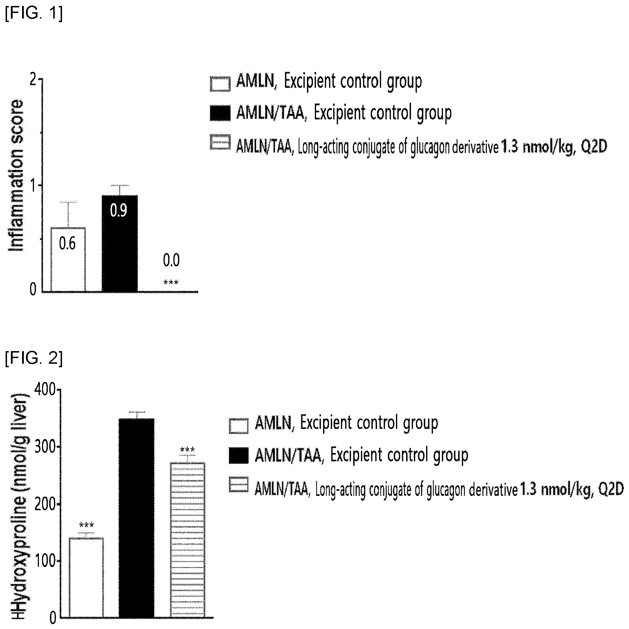

In an embodiment of the present invention, the effect of improving liver inflammation and liver fibrosis was confirmed in the AMLN (amylin)/TAA (Thioacetamide) mouse model (FIGS. 1 and 2), and this indicates that the glucagon derivative of the present invention can exhibit an effect in the major mechanism in the development of liver diseases including not only liver inflammation and liver fibrosis, but also primary liver cancer, which can be caused by hepatocytes mutated by liver inflammation, and liver cirrhosis, which is the last stage of liver fibrosis, and the resulting liver decompensation. The AMLN/TAA mouse model induced by administration of TAA (Thioacetamide), which is known to induce fibrosis through the induction of liver inflammation and AMLN diet, which is known to cause obesity and steatohepatitis due to its high fat, high fructose, and high cholesterol contents, is generally used as an inflammation or fibrosis model, and is further used as a non-alcoholic steatohepatitis model. Therefore, the glucagon derivative, a conjugate thereof, or a composition including the glucagon derivative according to the present invention can exhibit preventive or therapeutic effects on liver diseases including liver inflammation and liver fibrosis, and specifically, the liver disease may be caused by non-alcoholic steatohepatitis or may be accompanied thereby, but is not limited thereto.

As used herein, the term "liver inflammation", which is the most common cause of liver disease, refers to a disease that causes inflammation of the liver, and is divided into acute hepatitis and chronic hepatitis according to causes and symptoms. Viruses, alcohol, drugs, immune disorders, metabolic diseases, *etc.* are the main causes. When the composition of the present invention is administered, it may exhibit a preventive or therapeutic effect on liver inflammation by reducing the liver inflammation score in the administered subject, but is not limited thereto.

The peptide according to the present invention and a conjugate thereof not only have an effect of alleviating liver inflammation itself, but also exhibit effects on diseases accompanied or caused by liver inflammation.

As used herein, the term "fibrosis" is a disease that forms excessive fibrous connective tissues in an organ or tissue. Fibrosis refers to a state in which normal control is impossible during the wound healing process after the tissues in the human body are damaged by various stresses (inflammation, chemical stimulation, radiation, *etc.).* In particular, it is a pathway commonly encountered in chronic diseases, and its mechanism is very complex, so it has not been fully identified to date. Fibrosis occurs in various organs such as the lungs, heart, liver, *etc.,* and it is one of the areas with high unmet needs as no fundamental treatment has been developed yet.

As used herein, "liver fibrosis" refers to the result of a wound healing process for repeated liver damage, and is known to be reversible unlike cirrhosis, to be composed of thin fibrils, and to have no nodule formation. Once the cause of liver damage ceases, the recovery of normal liver may be possible. However, when the liver fibrosis process is repeated continuously, the crosslinking between the extra cellular matrices (ECMs) increases, thereby resulting in the progression of irreversible cirrhosis with nodules.

The composition of the present invention may exhibit a preventive or therapeutic effect on liver fibrosis by reducing the hydroxyproline content in the administered subject when the pharmaceutical composition is administered, but is not limited thereto. The peptide according to the present invention and a conjugate thereof not only have an effect of alleviating liver fibrosis itself, but also show effects on diseases accompanied or caused by liver fibrosis.

As used herein, the "liver cirrhosis" is a chronic disease that occurs with repeated regeneration of liver cells and an increase in fibrous tissue, it is pathologically accompanied by necrosis, inflammation, and fibrosis, and it ultimately progresses into cirrhosis complications (e.g., liver decompensation) and diseases (e.g., liver cancer), eventually leading to death. In particular, since liver cirrhosis can be discovered only after considerable progress due to the absence of awareness of one's own symptoms in the early stages of the disease, it is required that liver fibrosis, which is a condition before it evolves into cirrhosis, *etc.,* be treated promptly.

As used herein, the "liver decompensation" refers to a condition in which liver function is weakened and the liver cannot perform protein synthesis and metabolic functions as normal physiological functions due to viral hepatitis, cirrhosis, liver damage by drugs or alcohol, or liver disease. Liver decompensation is divided into acute liver decompensation and chronic liver decompensation according to the progression rate, and it is known to cause various complications.

As used herein, the "hepatocellular carcinoma" refers to a malignant tumor originating from liver cells, and it can be classified as primary liver cancer (hepatocellular carcinoma), which occurs in the liver cells themselves, and metastatic liver cancer, in which cancers of other tissues have metastasized to the liver, and about 90% or more of liver cancer is primary liver cancer. Major causes are alcohol, smoking, obesity, *etc.,* in addition to hepatitis and chronic liver disease.

As used herein, the "cholestasis" refers to a condition in which the bile flow from the liver to the duodenum is slowed or blocked, and "cholestasis liver disease" means that bile formation in the liver is impaired by conditions such as various diseases, expanded jugular nutrition, or side effects of specific drugs (*e.g*., some antibiotics). Common signs of cholestasis include fatigue, pruritus (itching), jaundice, and xanthoma (deposition of cholesterol-rich substances in the subcutaneous layer). The effects of cholestasis are extreme and broad, causing exacerbation of liver disease to a systemic disease, liver decompensation, and the need for liver transplantation. The causes of cholestasis liver disease may include acute hepatitis, inflammation of the bile ducts, *etc.*

The cholestasis liver disease may include primary biliary cholangitis (PBC), primary sclerosing cholangitis (PSC), progressive familial intrahepatic cholestasis (PFIC), and Alagille syndrome (AS), but the cholestasis liver disease is not limited thereto.

Primary biliary cirrhosis, which is also known as primary biliary cholangitis (PBC), is a cryptogenic chronic cholestasis liver disease. Progressive bile duct damage due to portal and periportal inflammation can cause progressive fibrosis and ultimate cirrhosis. Thus far, immunological, genetic, and environmental factors are known as potential causes of primary biliary cirrhosis. Primary biliary cirrhosis mostly occurs in middle-aged women, and symptoms such as fatigue, itching, or unidentified hyperlipidemia may also appear in the early onset of primary biliary cirrhosis.

At present, primary biliary cirrhosis is understood to be as an immune-mediated disease, and specifically, the immunohistochemical staining of T lymphocytes in the portal and periportal regions shows CD4-positive and CD8-negative T cells. In addition, abnormal suppressor T-cell activity was reported in asymptomatic first-grade relatives of affected subjects. It was reported that interleukins can have a role in the pathogenesis of PBC by contributing to altered immune functions and fibrosis (G. J. Webb et al., J. Autoimmunity, 2015 November; 64:42-52).

The method for treating PBC is a bile acid therapy using ursodeoxycholic acid (UDSA) and obeticholic acid (OCA). The action mechanism of the two drugs in PBC is associated with their ability to activate FXR and TGFR-5 to exert their anti-inflammatory effects. However, a sufficient biochemical response was not achieved in about 40% of the patients treated with UDCA.

Primary sclerosing cholangitis (PSC) is a chronic progressive cholestasis liver disease caused by cryptogenic inflammation and fibrosis of the intrahepatic and extrahepatic bile ducts. Specifically, it is an inflammatory disease of the bile ducts and biliary tract, and once the disease progresses, fibrosis occurs and the bile duct wall becomes thickened, thereby narrowing the bile ducts. The causes of the disease have not yet been identified, but it appears that a combination of various factors such as genetic factors, environmental factors, related immune responses, *etc.* may be a possible cause.

When the results of liver function tests through blood show an increase in alkaline phosphatase levels, an increase in aminotransferase levels, an indication of gamma globulinemia, *etc.,* the subject is diagnosed as having primary sclerosing cholangitis.

The method for treating PSC has not been clearly reported thus far, and liver transplant surgery is the only treatment that can treat PSC fundamentally.

Accordingly, there is still a need to develop a drug capable of treating PBS and PSC without side effects while securing the patient's convenience.

The composition of the present invention exhibits (a) reduction in a liver inflammation score; (b) the effect of reducing the hydroxyproline content; and/or (c) reduction in triglyceride content in liver tissue, thereby preventing or treating liver disease, but is not limited thereto.

The composition of the present invention includes a glucagon derivative or a conjugate thereof having preventive, ameliorative, or therapeutic effects on simple steatosis, liver inflammation, and liver fibrosis, and thus can be used for preventing or treating liver disease. The composition of the present invention can be administered to a subject having the above-described liver disease, which can be improved or treated by administration of the peptide according to the present invention, to exhibit a prevention or treatment effect on liver disease. In addition, the subject to which the composition of the present invention is administered may be a subject having non-alcoholic steatohepatitis or a liver disease caused thereby, but is not limited thereto. As used herein, the term "prevention" refers to all activities that inhibit or delay the occurrence of liver disease by administering the glucagon derivative, a conjugate including the same, or the composition, and the term "treatment" refers to all activities that improve or advantageously change the symptoms of liver disease by administering the glucagon derivative, a conjugate including the same, or the composition.

As used herein, the term "administration" refers to the introduction of a particular material into a patient by any appropriate method, and the administration route of the composition is not particularly limited, but may be any conventional route that enables delivery of the composition to the target in the body (*e.g*., intraperitoneal administration, intravenous administration, intramuscular administration, subcutaneous administration, intradermal administration, oral administration, topical administration, intranasal administration, intrapulmonary administration, intrarectal administration, *etc.).*

The glucagon derivative according to the present invention has an altered pl as compared to native glucagon, and thus can exhibit improved solubility and higher stability at a neutral pH condition in the form of a long-acting conjugate. Additionally, the glucagon derivative according to the present invention can exhibit an activity on a glucagon receptor and thus can be effectively used for preventing or treating target diseases including liver disease.

The pharmaceutical composition of the present invention may contain a pharmaceutically acceptable carrier, excipient, or diluent. As used herein, the term "pharmaceutically acceptable" refers to the properties of having a sufficient amount to exhibit a therapeutic effect and not causing adverse effects, and may be easily determined by a skilled person in the art based on the factors well known in the medical field, such as the kind of disease, age, body weight, health status, sex, drug sensitivity of a patient, administration route, administration method, administration frequency, duration of treatment, a drug to be mixed or administered simultaneously in combination, *etc.*

The pharmaceutical composition of the present invention containing the peptide or a conjugate thereof may further contain a pharmaceutically acceptable excipient. The pharmaceutically acceptable excipient may include, for oral administration, a binder, a lubricant, a disintegrant, a solubilizing agent, a dispersant, a stabilizing agent, a suspending agent, a coloring agent, a fragrance, *etc*.; for injections, a buffering agent, a preservative, an analgesic, a solubilizing agent, an isotonic agent, a stabilizing agent, *etc.,* which may be combined to be used; and for topical administrations, a base, an excipient, a lubricant, a preservative, *etc.,* although it is not particularly limited thereto.

The formulation type of the composition according to the present invention may be prepared variously by being combined with a pharmaceutically acceptable excipient described above. For example, for oral administration, the composition may be formulated into tablets, troches, capsules, elixirs, suspensions, syrups, wafers, *etc.,* and for injections, the composition may be formulated into unit-dose ampoules or multi-dose containers. The composition may also be formulated into solutions, suspensions, tablets, pills, capsules, sustained-release formulations, *etc.*

Meanwhile, examples of suitable carriers, excipients, and diluents for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anti-coagulant, a lubricant, a humectant, a fragrance, a preservative, *etc.*

Additionally, the pharmaceutical composition of the present invention may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated into a preparation of a unit dosage form suitable for the administration into a patient's body, and may specifically be formulated into a preparation useful for peptide drugs according to the conventional method in the pharmaceutical field so as to be administered by an oral or parenteral route (including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal route), but the administration routes are not limited thereto.

Additionally, the peptide or conjugate may be used by being mixed with various pharmaceutically acceptable carriers such as physiological saline or organic solvents. To increase stability or absorptivity, carbohydrates (*e.g*., glucose, sucrose, or dextrans), antioxidants (*e.g*., ascorbic acid or glutathione), chelating agents, low-molecular weight proteins, or other stabilizers, *etc.* may be used as pharmaceutical drugs.

The administration dose and frequency of the pharmaceutical composition of the present invention are determined by the type of drugs, which are active ingredients, together with various factors, such as the disease to be treated, administration route, patient's age, sex, and body weight, severity of the disease, *etc.* Specifically, the composition of the present invention may be one which contains the peptide or a long-acting conjugate including the peptide in a pharmaceutically effective amount, but the composition of the present invention is not limited thereto.

Containing the peptide or a long-acting conjugate thereof in a pharmaceutically effective amount refers to a level at which the desired pharmacological activity (*e.g*., prevention, improvement, or treatment of liver disease) can be obtained by the peptide or a long-acting conjugate thereof, and in addition, may refer to a pharmaceutically acceptable level, which is a level at which toxicities or adverse effects do not occur or occur at an insignificant level in the subject to be administered, but the level is not limited thereto. The pharmaceutically effective amount may be determined by comprehensively considering the number of administration, patient, formulations, *etc.*

Although not particularly limited thereto, the pharmaceutical composition of the present invention may contain the above ingredients (active ingredients) in an amount of 0.01 % (w/v) to 99% (w/v).

The total effective dose of the composition of the present invention may be administered to a patient in a single dose or may be administered for a long period of time in multiple doses according to a fractionated treatment protocol. In the pharmaceutical composition of the present invention, the content of active ingredient(s) may vary depending on the severity of the disease. Specifically, the total daily dose of the peptide or conjugate of the present invention may be about 0.0001 µg to 500 mg per 1 kg of the body weight of a patient. However, the effective dose of the peptide or conjugate is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the pharmaceutical composition of the present invention. The pharmaceutical composition according to the present invention is not particularly limited to the formulation type and administration route and mode, as long as it shows the effects of the present invention.

The pharmaceutical composition of the present invention may have excellent *in vivo* duration of efficacy and titer, and thus, the number and frequency of administration of the pharmaceutical preparation of the present invention can be significantly reduced, but is not particularly limited thereto.

Another aspect for implementing the present invention provides a pharmaceutical composition for preventing or treating liver disease, including a peptide including any one amino acid sequence of SEQ ID NOS: 20, 22, 33, 37, and 38, or a conjugate thereof.

The liver disease may be of steatosis, liver fibrosis, liver inflammation, liver cirrhosis, liver decompensation, hepatocellular carcinoma, and cholestasis liver disease, and the cholestasis liver disease may be primary biliary cirrhosis, primary sclerosing cholangitis, and a combination thereof, but the liver disease is not limited thereto. Additionally, the liver disease may be caused by non-alcoholic steatohepatitis (NASH) or accompanied thereby, but is not limited thereto.

The peptide, conjugate, liver disease, prevention, treatment, and pharmaceutical composition are as described above.

Still aspect for implementing the present invention provides a method for preventing or treating liver disease, including administering the peptide, a conjugate including the same, or a composition including the same to a subject.

The peptide, conjugate including the same, composition including the same, liver disease, prevention, and treatment are as described above.

As used herein, the subject refers to a subject suspected of having a liver disease, and the subject suspected of having a liver disease refers to mammals including humans, rats, livestock, *etc.* which have or are at risk of developing the liver disease, but any subject which can be treated with the glucagon derivative of the present invention or the composition including the same is included without limitation. Additionally, by administering the pharmaceutical composition including the glucagon derivative of the present invention to a subject suspected of having liver disease, the subject can be efficiently treated. The liver disease is as described above.

Specifically, the subject suspected of having liver disease may be a subject having non-alcoholic steatohepatitis (NASH), or a subject having steatosis, liver fibrosis, liver inflammation, liver cirrhosis, liver decompensation, hepatocellular carcinoma, cholestasis liver disease, primary biliary cirrhosis, or primary sclerosing cholangitis caused by NASH, but is not limited thereto. The method of the present invention may include administering the pharmaceutical composition including the peptide in a pharmaceutically effective amount. An appropriate total daily dose of the pharmaceutical composition may be determined within the scope of correct medical judgment by a practitioner, and the pharmaceutical composition may be administered once or several times in divided doses. However, for the purpose of the present invention, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, various factors including drugs used in combination or simultaneously with the specific compositions, and similar factors well known in the medical field.

Yet another aspect for implementing the present invention provides the use of the peptide, a conjugate including the same, or the composition in the preparation of a medicament for preventing or treating liver disease (pharmaceutical composition).

The peptide, conjugate, composition, and liver disease are as described above.

Even another aspect for implementing the present invention provides the use of the peptide, a conjugate including the same, or the composition in the prevention or treatment of liver disease.

The peptide, conjugate, composition, and liver disease are as described above.

Hereinafter, the present invention will be described in more detail with reference to the following Examples. However, these Examples are for illustrative purposes only and the scope of the invention is not limited by these Examples.

### Example 1: Production of Cell Line Showing cAMP Response to Glucagon

PCR was performed using a region corresponding to Open Reading Frame (ORF) in the cDNA (OriGene Technologies, Inc., USA) of human glucagon receptor gene as a template along with the following forward and reverse primers (SEQ ID NOS: 47 and 48, respectively), which include each of the *EcoR*I and *Xho*I restriction sites.

In particular, PCR was performed for a total of 30 cycles under the following conditions: 95°C denaturation for 60 sec, annealing at 55°C for 60 sec, and polymerization at 68°C for 30 sec. The amplified PCR products were subjected to a 1.0% agarose gel electrophoresis, and a 450 bp band was obtained by elution.
Forward primer (SEQ ID NO: 47):
   5'-CAGCGACACCGACCGTCCCCCCGTACTTAAGGCC-3'
Reverse primer (SEQ ID NO: 48):
   5'-CTAACCGACTCTCGGGGAAGACTGAGCTCGCC-3'

The PCR product was cloned into a known animal cell expression vector, x0GC/dhfr, to prepare a recombinant vector x0GC/GCGR.

CHO DG44 cell line cultured in DMEM/F12 medium containing 10% FBS was transfected with the prepared recombinant vector x0GC/GCGR using Lipofectamine^{®}, and cultured in a selection medium containing G418 (1 mg/mL) and methotraxate (10 nM). Single clone cell lines were selected therefrom by a limit dilution technique, and a cell line showing excellent cAMP response to glucagon in a concentration-dependent manner was finally selected therefrom.

### Example 2: Synthesis of Glucagon Derivatives

In order to prepare glucagon derivatives with improved physical properties, the amino acid sequence of native glucagon of SEQ ID NO: 1 was substituted with amino acid residues having positive and negative charges, and thereby glucagon derivatives were synthesized as shown in Table 1 below. The relative *in vitro* activities described below were measured by the method described in Example 4.

**[Table 1]**

| SEQ ID NO: | Glucagon Analog Sequences | Ring Formation | pI | *In vitro* Activity (relative activity to SEQ ID NO: 1, %) |
|---|---|---|---|---|
| SEQ ID NO: 1 | HSQGTFTSDYSKYLDSRRAQDFVQW LMNT | - | 6.8 | 100 |
| SEQ ID NO: 2 | HSQGTFTSDYSKYLDCDRAQDFVQW LMNT | - | 4.56 | 0.6 |
| SEQ ID NO: 3 | HSQGTFTSDYSKYLDCERAQDFVQW LMNT | - | 4.66 | 6.1 |
| SEQ ID NO: 4 | HSQGTFTSDYSKYLDSCDAQDFVQW LMNT | - | 4.13 | < 0.1 |
| SEQ ID NO: 5 | HSQGTFTSDYSKYLDSCEAQDFVQWL MNT | - | 4.22 | 0.3 |
| SEQ ID NO: 6 | HSQGTFTSDYSKYLDSCEADDFVQWL MNT | - | 4.03 | < 0.1 |
| SEQ ID NO: 7 | YSQGTFTSDYSKYLDSCEADDFVQWL MNT | - | 3.71 | < 0.1 |
| SEQ ID NO: 8 | YXQGTFTSDYSKYLDSCDAQDFVQWL INT | - | 3.77 | < 0.1 |
| SEQ ID NO: 9 | YXQGTFTSDYSKYLDSCDAQDFVVWL INT | - | 3.77 | < 0.1 |
| SEQ ID NO: 10 | YXQGTFTSDYSKYLDSCDADDFVVWL INT | - | 3.66 | < 0.1 |
| SEQ ID NO: 11 | YXQGTFTSDYSKYLDEKCAKEFVQWL MNT | - | 4.78 | 4.6 |
| SEQ ID NO: 12 | YXQGTFTSDYSKYLDEKRAKEFVQWL MNTC | ring formed | 6.20 | 56.3 |
| SEQ ID NO: 13 | YXQGTFTSDYSCYLDSRRAQDFVQW LMNT | - | 4.43 | 5.2 |
| SEQ ID NO: 14 | YXQGTFTSDYSKYLDCKRAKEFVQWL MNT | - | 8.12 | 18.1 |
| SEQ ID NO: 15 | YXQGTFTSDYSKYLCEKRAQDFVVWL [?] MNT | - | 6.11 | 1.1 |
| SEQ ID NO: 16 | YXQGTFTSDYSKYLDCRRAQVFVQWL - MRT | - | 9.11 | 4.2 |
| SEQ ID NO: 17 | YXQGTFTSDYSKYLDCVRAQDFVQWL [?] MRT | - | 6.03 | 23.2 |
| SEQ ID NO: 18 | YXQGTFTSDYSKYLDSRRACDFRLWL - MNT | - | 8.15 | < 0.1 |
| SEQ ID NO: 19 | YXQGTFTSDYSKYLCEKRAKEFVQWL MNT | ring formed | 8.12 | 12.1 |
| SEQ ID NO: 20 | YXQGTFTSDYSKYLDECRAKEFVQWL MNT | ring formed | 4.78 | 299.7 |
| SEQ ID NO: 21 | YXQGTFTSDYSKYLDEKCAKEFVQWL MNT | ring formed | 4.78 | 57.8 |
| SEQ ID NO: 22 | YXQGTFTSDYSKYLDEKRCKEFVQWL MNT | ring formed | 6.20 | 147.8 |
| SEQ ID NO: 23 | YXQGTFTSDYSKYCDEKRAKEFVQWL MNT | ring formed | 6.20 | 76.8 |
| SEQ ID NO: 24 | YXQGTFTSDYSKCLDEKRAKEFVQWL MNT | ring formed | 6.21 | 58.0 |
| SEQ ID NO: 25 | YXQGTFTSDYSKYLDEKRAKCFVQWL MNT | ring formed | 8.12 | 46.9 |
| SEQ ID | WXQGTFTSDYSKYLDECRAKDFVQW | ring | 4.68 | 1.0 |
| NO: 26 | LMNT | formed | | |
| SEQ ID NO: 27 | YXQGTFVSDYSKYLDECRAKDFVQWL MNT | ring formed | 4.68 | 93.6 |
| SEQ ID NO: 28 | WXQGTFVSDYSKYLDECRAKDFVQW LMNT | ring formed | 4.68 | < 0.1 |
| SEQ ID NO: 29 | YXQGTFTSDYSKCLDERRAKDFVQWL MNT | ring formed | 6.15 | 61.3 |
| SEQ ID NO: 30 | WXQGTFTSDYSKCLDERRAKDFVQW LMNT | ring formed | 4.44 | 0.3 |
| SEQ ID NO: 31 | YXQGTFTSDYSKYLDCKRAKEFVQWL MNT | ring formed | 8.12 | 6.3 |
| SEQ ID NO: 32 | -SQGTFTSDYSKYLDECRAKEFVQWL MNT | ring formed | 4.78 | 0.7 |
| SEQ ID NO: 33 | YXQGTFTSDYSKYLDSRRAQDFVQWL MNT | - | 6.04 | 108.2 |
| SEQ ID NO: 34 | WXQGTFTSDYSKYCDERRAKEFVQW LMNT | ring formed | 6.21 | 0.2 |
| SEQ ID NO: 35 | YXQGTFTSDYSKYCDERRAKEFVQW LMNT | ring formed | 6.2 | 17.7 |
| SEQ ID NO: 36 | YXQGTFTSDCSKYLDERRAKEFVQWL MNT | ring formed | 6.21 | 9.9 |
| SEQ ID NO: 37 | YXQGTFTSDYSKYLDERRAKEFVQWL MNTC | ring formed | 6.21 | 225.5 |
| SEQ ID NO: 38 | YXQGTFCSDYSKYLDERRAKEFVQWL MNT | ring formed | 6.15 | 167.3 |
| SEQ ID NO: 39 | YXQGTFVSDCSKYLDERRAKDFVQW LMNT | ring formed | 6.15 | 3.7 |
| SEQ ID NO: 40 | YXQGTFVSDYSKYLDERRAKDFVQWL MNTC | ring formed | 6.15 | 40.8 |
| SEQ ID NO: 41 | YXQGTFCSDYSKYLDERRAKDFVQW LMNT | ring formed | 6.03 | 45.2 |
| SEQ ID NO: 42 | YXQGTFCSDYSKYLDSRRAQDFVQW LMNT | - | 6.03 | 37.9 |
| SEQ ID NO: 43 | YXQGTFTSDCSKYLDSRRAQDFVQW LMNT | - | 6.03 | 1.6 |
| SEQ ID NO: 44 | YXQGTFTSDYSKYLDSRRAQDFVQWL MNTC | - | 6.21 | 75.4 |
| SEQ ID NO: 45 | YXQGTFTSDYSCYLDEKRAKEFVQWL MNT | - | 4.78 | 5.2 |

In the amino acids sequences described in Table 1, the amino acid represented by X represents a non-native amino acid, aminoisobutyric acid (Aib), the underlined amino acid residues represent formation of a lactam ring between the side chains of the corresponding amino acids, and "-" in the amino acid sequence indicates that no amino acid residue is present on the corresponding position. Additionally, in the rows with regard to ring formation, "-" indicates that there is no ring formation in the corresponding sequences.

### Example 3: Measurement of pl of Glucagon Derivatives

In order to measure the improved physical properties of glucagon derivatives synthesized in Example 2, pl values were calculated based on the amino acid sequences using the pI/Mw tool (http://expasy.org/tools/pi_tool.html; Gasteiger *et al.,* 2003) in the ExPASy server.

As shown in Table 1 above, while the native glucagon of SEQ ID NO: 1 had a pl of 6.8, some glucagon derivatives according to the present invention showed pl values in the range of from about 4 to about 6. Since the glucagon derivatives according to the present invention have pl values lower or higher than that of native glucagon, they can exhibit improved solubility and higher stability at a neutral pH condition compared to native glucagon.

### Example 4: Measurement of cAMP Activity of Glucagon Derivatives

The activities of the glucagon derivatives synthesized in Example 2 were measured in cell lines having the human glucagon receptors produced in Example 1. Specifically, the transfected cell line was sub-cultured 3 to 4 times a week, aliquoted into a 384-well plate in an amount of 6×10³ cell lines/well, and cultured for 24 hours. Native glucagon and glucagon derivatives were suspended in Hank's balanced salt solution (HBSS) buffer containing 0.5 mM of 3-isobutyl-1-methylxanthine (IBMX), 0.1% bovine serum albumin (BSA), and 5 mM 4-(2-hydroxyethyl)-1-piperazineethanesulfonic acid (HEPES) with the culture cells, at concentrations of 200 nM and 1600 nM, respectively, and then continuously subjected into a 4-fold dilution 10 times, applied to a cAMP assay kit (LANCE cAMP 384 kit, PerkinElmer), and added to the cultured cells, and their fluorescence values were measured. Upon measurement, the highest fluorescence value was set at 100% and then EC₅₀ values of the glucagon derivative were calculated based on the same and compared with that of native glucagon, respectively. The results are shown in Table 1 above.

### Example 5: Preparation of Long-Acting Conjugate of Glucagon Derivatives

The long-acting conjugate of the glucagon derivative peptides was prepared in the following manner:
Maleimide-PEG-aldehyde (Japan NOF Inc.,), which is a linearly modified polyethylene glycol with a molecular weight of 10 kDa, in which the hydrogens at both terminals are substituted with a 3-(3-maleimidopropionamido)propyl group and a 3-oxopropyl group (propionaldehyde group), respectively, was allowed to react with the derivatives with a cysteine residue among the above-described glucagon derivative peptides to PEGylate the cysteine residue of the glucagon derivative peptide at the maleimide end of maleimide-PEG-aldehyde. Specifically, the glucagon derivative peptide of SEQ ID NO: 37 and maleimide-PEG-aldehyde were reacted at a molar ratio of 1:1 to 5, at a protein concentration of 3 mg/mL to 10 mg/mL at low temperature for 1 to 3 hours. In particular, the reaction was conducted in an environment in which 20% to 60% isopropanol was added to 50 mM Tris buffer (pH 7.5). Upon completion of the reaction, the reaction solutions were applied to SP sepharose HP (GE Healthcare, USA) to purify the glucagon derivative mono-PEGylated on cysteine.

The immunoglobulin Fc region was prepared using the immunoglobulin Fc region (49.8 kDa, a homodimer in which two chains of SEQ ID NO: 69 are linked by a disulfide bond) having a hinge region of the Pro-Ser-Cys-Pro sequence (SEQ ID NO: 59) at the N-terminus by the method described in International Patent Publication No. WO 2007/021129.

Then, the purified mono-PEGylated glucagon derivative peptide and the immunoglobulin Fc fragment were reacted at a molar ratio of 1:2 to 10 at a protein concentration of 10 mg/mL to 50 mg/mL at 4°C to 8°C for 12 to 18 hours. The reaction was performed in an environment in which 10 mM to 50 mM sodium cyanoborohydride and 10% to 20% isopropanol were added to 100 mM potassium phosphate butter (pH 6.0). Upon completion of the reaction, the reaction solutions were applied to the Butyl Sepharose FF purification column (GE Healthcare, USA) and Source ISO purification column (GE Healthcare, USA) to purify the long-acting conjugate of the glucagon derivative peptide in which the polyethylene glycol end at the aldehyde side of the mono-PEGylated glucagon derivative peptide is linked to the nitrogen at the N-terminal proline of one of the two chains of the immunoglobulin Fc homodimer.

After the preparation, the purity analyzed by reverse phase chromatography, size exclusion chromatography, and ion exchange chromatography was shown to be 95% or more.

In particular, the conjugate, in which the glucagon derivate peptide and the immunoglobulin Fc fragment are linked through PEG, was designated as the "long-acting conjugate of glucagon derivative".

### Example 6: Effect of Improving Liver Inflammation of Long-Acting Conjugate of Glucagon Derivative

In order to confirm the effect of improvement of liver inflammation by the long-acting conjugate prepared in Example 5, an AMLN/TAA (amylin/thioacetamide) mouse model was used. C57BL/6 mice were subjected to AMLN dietary intake and TAA administration (50 mg/kg to 400 mg/kg, TIW: 3 times per week) for 10 weeks to induce a model.

The induced AMLN/TAA mouse model was divided into an excipient control group and a group administered with the long-acting conjugate (1.3 nmol/kg, Q2D), and was repeatedly administered subcutaneously for 4 weeks. The mice fed with only an AMLN diet were used as a negative control. After 4 weeks of repeated administration, H&E staining was performed on the liver tissue of each mouse taken through necropsy, and the liver inflammation score was measured.

As shown in FIG. 1, it was confirmed that as a result of the repeated administration of the long-acting conjugate for 4 weeks, the liver inflammation score was significantly reduced in the group administered with the long-acting conjugate compared to the AMLN/TAA excipient control group (***p < 0.001 vs. AMLN/TAA excipient control group by one-way ANOVA). Through the reduction of the liver inflammation score, it was confirmed that the long-acting conjugate of the glucagon derivative had an effect of improving liver inflammation.

### Example 7: Effect of Improving Liver Fibrosis of Long-Acting Conjugate of Glucagon Derivative

In order to confirm the effect of improving liver fibrosis by the long-acting conjugate prepared in Example 5, an AMLN/TAA (amylin/thioacetamide) mouse model was used as in Example 6. The induced AMLN /TAA mouse model was divided into an excipient control group and a group administered with the long-acting conjugate (1.3 nmol/kg, Q2D), and was repeatedly administered subcutaneously for 4 weeks. The mice fed with only an AMLN diet were used as a negative control. After 4 weeks of repeated administration, the content of hydroxyproline, which is a marker of invasive fibrosis, was measured in the liver tissue of each mouse taken through necropsy.

As shown in FIG. 2, in the AMLN/TAA excipient control group, the content of hydroxyproline in liver tissue was increased, whereas in the group administered with the long-acting conjugate, the content of hydroxyproline in liver tissue was significantly reduced by administration of the long-acting conjugate (***p < 0.001 vs. AMLN/TAA excipient control group by one-way ANOVA). It was confirmed that the long-acting conjugate of the glucagon derivative had an effect of improving liver fibrosis through the improvement of the invasive fibrosis index.

### Example 8: Effect of Improving Steatosis of Long-Acting Conjugate of Glucagon Derivative

In order to confirm the effect of improving simple steatosis by the long-acting conjugate of glucagon derivative prepared in Example 5, AMLN mice, an animal model in which non-alcoholic fatty liver was induced by induction of a high-fat diet, were used. Non-alcoholic fatty liver was induced by AMLN dietary intake (40 kcal-% fat, 22% fructose, 10% sucrose, 2% cholesterol) in C57BL/6 mice for 29 weeks. The mouse model was divided into normal mice fed with normal diet, mice with non-alcoholic fatty liver disease induced by AMLN diet (excipient control group), a group administered with the long-acting conjugate of glucagon derivative (2.0 nmol/kg, Q2D), and the substance was repeatedly administered subcutaneously for 12 weeks. After repeated administration, the liver tissue of each mouse was taken through necropsy, and the degree of improvement in steatosis by the long-acting conjugate of the glucagon derivative was evaluated by the level of triglyceride in the liver.

FIG. 3 shows the results of evaluating the level of triglyceride per gram of liver in each group. As a result of repeated administration of the long-acting conjugate of the glucagon derivative for 12 weeks, it was confirmed that the level of triglyceride in the liver tissue was significantly decreased when compared to the excipient control group (normal diet intake group = 15.0 mg of triglyceride per gram of liver, AMLN diet control group = 139.0 mg per gram of liver, AMLN diet long-acting conjugate of glucagon derivative 2.0 nmol/kg = 25.0 mg of triglyceride per gram of liver).

For statistical treatment, the efficacy of the long-acting conjugate of glucagon derivative was evaluated by comparing with the excipient control group using a one-way ANOVA (*p<0.05, **p<0.01, and ***p<0.001)

It can be expected through such results that steatosis, liver inflammation, and liver fibrosis can be effectively improved upon administration of the long-acting conjugate of the glucagon derivative of the present invention, suggesting that further improvement can be made to other liver diseases caused by steatosis, liver inflammation, and liver fibrosis.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A pharmaceutical composition for preventing or treating liver disease, comprising a pharmaceutically acceptable excipient; and a peptide comprising the amino acid sequence of General Formula 1 below in a pharmaceutically effective amount:
Y-X2-QGTF-X7-SDYSKY-X14-D-X16-X17-R-X19-X20-X21-FVQWLMNT-X30 (General Formula 1, SEQ ID NO: 46)
wherein,
X2 is aminoisobutyric acid (Aib);
X7 is threonine (T), valine (V), or cysteine (C);
X14 is leucine (L) or cysteine (C);
X16 is glutamic acid (E) or serine (S);
X17 is lysine (K), arginine (R), or cysteine (C);
X19 is alanine (A) or cysteine (C);
X20 is lysine (K) or glutamine (Q);
X21 is aspartic acid (D) or glutamic acid (E); and
X30 is cysteine (C) or is absent (with the proviso that when the amino acid sequence of General Formula 1 is identical to SEQ ID NO: 1 or SEQ ID NO: 12, it is excluded).

2. The pharmaceutical composition of claim 1, wherein the peptide is in the form of a long-acting conjugate, and the long-acting conjugate is represented by Chemical Formula 1 below:
[Chemical Formula 1] X-L-F
with the proviso that X is a peptide comprising the amino acid sequence of General Formula 1 above;
L is a linker containing ethylene glycol repeating units;
F is an immunoglobulin Fc region; and
- represents a covalent bond between X and L, and L and F.

3. The pharmaceutical composition of claim 1 or 2, wherein the liver disease is any one selected from the group consisting of steatosis, liver fibrosis, liver inflammation, liver cirrhosis, liver decompensation, hepatocellular carcinoma, and cholestasis liver disease.

4. The pharmaceutical composition of claim 3, wherein the cholestasis liver disease is any one selected from the group consisting of primary biliary cirrhosis, primary sclerosing cholangitis, and a combination thereof.

5. The pharmaceutical composition of claim 3, wherein the liver disease is caused by non-alcoholic steatohepatitis (NASH) or is accompanied thereby.

6. The pharmaceutical composition of claim 1 or 2, wherein the peptide comprises an amino acid sequence selected from SEQ ID NOS: 20, 22, 23, 27, 33, 35, 37, 38, 40, 41, 42, and 44.

7. The pharmaceutical composition of claim 1 or 2, wherein the peptide comprises an amino acid sequence selected from SEQ ID NOS: 20, 22, 23, 27, 33, 37, 38, and 44.

8. The pharmaceutical composition of claim 1 or 2, wherein the pharmaceutical composition reduces a liver inflammation score in the administered subject upon administration.

9. The pharmaceutical composition of claim 1 or 2, wherein the pharmaceutical composition reduces hydroxyproline content in the administered subject upon administration.

10. The pharmaceutical composition of claim 1 or 2, wherein the pharmaceutical composition reduces triglyceride content in the liver tissue in the administered subject upon administration.

11. The pharmaceutical composition of claim 1 or 2, wherein the C-terminus of the peptide is amidated.

12. The pharmaceutical composition of claim 2, wherein the formula weight of the ethylene glycol repeating unit moiety in L is in the range of 1 kDa to 100 kDa.

13. A pharmaceutical composition for preventing or treating liver disease, comprising a pharmaceutically acceptable excipient; and
a peptide comprising any one of amino acid sequences of SEQ ID NOS: 20, 22, 33, 37, and 38.

14. The pharmaceutical composition of claim 13, wherein the peptide is in the form of a long-acting conjugate, and the long-acting conjugate is represented by Chemical Formula 1 below:
[Chemical Formula 1] X-L-F
with the proviso that X is a peptide comprising any one of amino acid sequences of SEQ ID NOS: 20, 22, 33, 37, and 38;
L is a linker containing ethylene glycol repeating units;
F is an immunoglobulin Fc region; and
- represents a covalent bond between X and L, and L and F.

15. The pharmaceutical composition of claim 13 or 14, wherein the liver disease is any one selected from the group consisting of steatosis, liver fibrosis, liver inflammation, liver cirrhosis, liver decompensation, hepatocellular carcinoma, and cholestasis liver disease.

16. The pharmaceutical composition of claim 15, wherein the cholestasis liver disease is any one selected from the group consisting of primary biliary cirrhosis, primary sclerosing cholangitis, and a combination thereof.

17. The pharmaceutical composition of claim 15, wherein the liver disease is caused by non-alcoholic steatohepatitis (NASH) or is accompanied thereby.
